# EUROPÄISCHE PATENTSCHRIFT

(11) **EP 3 121 181 B1**
(45) Veröffentlichungstag und Bekanntmachung des Hinweises auf die Patenterteilung: **28.11.2018**
(21) Anmeldenummer: 16180043.8
(22) Anmeldetag: 19.07.2016
(51) Int. Cl.: B01J 31/22, B01J 31/24, C07C 67/38, C07F 17/02, C07F 9/572, C07F 9/655, C07F 9/6553, C07F 15/00, C07F 9/58, C07F 9/6506

(54) **MONOPHOSPHINVERBINDUNGEN UND DARAUF BASIERENDE PALLADIUM-KATALYSATOREN FÜR DIE ALKOXYCARBONYLIERUNG ETHYLENISCH UNGESÄTTIGTER VERBINDUNGEN**
MONOPHOSPHINE COMPOUNDS AND PALLADIUM-CATALYSTS BASED ON SAME FOR THE ALKOXYCARBONYLATION OF ETHYLENICALLY UNSATURATED COMPOUNDS
COMPOSÉS A BASE DE MONO-PHOSPHINE ET CATALYSEURS EN PALLADIUM A BASE DE CELLES-CI POUR L'ALKOXY-CARBONYLATION DE COMPOSÉS INSATUREES EN ETHYLENE

(30) Priorität: 23.07.2015 DE 102015213918
(43) Veröffentlichungstag der Anmeldung: 25.01.2017
(73) Patentinhaber: Evonik Degussa GmbH, 45128 Essen (DE)
(72) Erfinder: DONG, Kaiwu, 236800 Bo Zhou (CN); JACKSTELL, Ralf, 27478 Cuxhaven Altenwalde (DE); BELLER, Matthias, 18211 Ostseebad Nienhagen (DE); FRANKE, Robert, 45772 Marl (DE); HESS, Dieter, 45770 Marl (DE); DYBALLA, Katrin Marie, 45657 Recklinghausen (DE); FRIDAG, Dirk, 45721 Haltern am See (DE); GEILEN, Frank, 45721 Haltern am See (DE)

(56) Entgegenhaltungen:
- EP-A1- 0 386 834
- EP-A1- 0 441 446
- EP-A1- 0 441 447
- DE-A1- 4 141 299
- R X.-B M P.E J.N.H Lemmens Jang Kuil Leeuwen Gouiaan Reek ET AL: "Synthesis of building blocks for the development of the SUPRAPhos ligand library and examples of their application in catalysis", European Journal of Organic Chemistry, 1. Januar 2008 (2008-01-01), Seiten 6079-6092, XP55134445, DOI: 10.1002/ejoc.200800499 Gefunden im Internet: URL:http://dare.uva.nl/record/304117
- LEONID I. GORYUNOV ET AL: "Compounds R1R2EMMe3 (E = P, As; M = Si, Sn) - Convenient and Versatile Reagents for the Syntheses of Tertiary (Fluoroaryl)phosphanes and -arsanes", COLLECTION OF CZECHOSLOVAK CHEMICAL COMMUNICATIONS, Bd. 73, Nr. 12, 1. Januar 2008 (2008-01-01), Seiten 1612-1622, XP55262909, PRAGUE; CZ ISSN: 0010-0765, DOI: 10.1135/cccc20081612
- NATALIA F. BLANK ET AL: "Palladium-Catalyzed Asymmetric Phosphination. Scope, Mechanism, and Origin of Enantioselectivity", JOURNAL OF THE AMERICAN CHEMICAL SOCIETY, Bd. 129, Nr. 21, 1. Mai 2007 (2007-05-01), Seiten 6847-6858, XP55322344, US ISSN: 0002-7863, DOI: 10.1021/ja070225a
- MELINDA J. GREEN ET AL: "Palladium(ii) complexes of new OPN phosphine ligands and their application in homogeneously catalysed reactions of CO with alkenes or alkynes", DALTON TRANSACTIONS: THE INTERNATIONAL JOURNAL FOR INORGANIC, ORGANOMETALLIC AND BIOINORGANIC CHEMISTRY, Nr. 20, 1. Januar 2004 (2004-01-01), Seite 3251, XP55322350, GB ISSN: 1477-9226, DOI: 10.1039/b405586c
- SHAN-MING KUANG ET AL: "Formation of donor-acceptor Fe(0)-Hg(II) bond in separation and stabilization of optically active iron(0) phosphine complexes. Absolute configuration of (+)-(R)-(CO)4Fe([mu]-EtPhPpy)HgCl2", INORGANICA CHIMICA ACTA, Bd. 293, Nr. 1, 1. Oktober 1999 (1999-10-01), Seiten 106-109, XP55322354, NL ISSN: 0020-1693, DOI: 10.1016/S0020-1693(99)00216-9
- KUANG SHAN-MING ET AL: "Coordination chemistry of organometallic polydentate ligands. syntheses of Fe-M complexes using Fe(CO)4(Ph2Ppy-P)[Ph2Ppy = 2-(Diphenylphosphino)Pyridine] and TRANS-Fe(PhPMepy)2(CO)3[PhPMepy = 2-(Phenylmethylphosphino) Pyridine] AS A Neutral BI- OR Tridentate Ligand", POLYHEDRON, Bd. 15, Nr. 19, 1996, Seiten 3417-3426, XP028698911, ISSN: 0277-5387, DOI: 10.1016/0277-5387(96)00059-9

## Beschreibung

Die vorliegende Erfindung betrifft neue Monophosphinverbindungen und deren Verwendung in der Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung von ethylenisch ungesättigten Verbindungen, wie Olefinen, mit Kohlenmonoxid und Alkoholen in Gegenwart eines Metalls bzw. eines Metallkomplexes und eines Liganden zu den entsprechenden Estern:

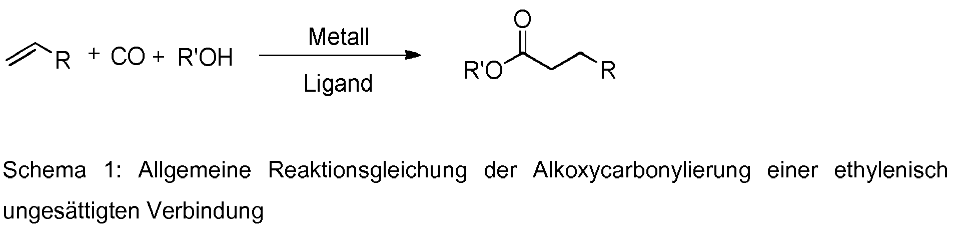

Unter den Alkoxycarbonylierungsreaktionen ist die Ethen-Methoxycarbonylierung zu 3-Methylpropionat von Bedeutung als Zwischenstufe für die Herstellung von Methylmethacrylat (S. G. Khokarale, E. J. García-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt.

Die Alkoxycarbonylierung kann zu verzweigten (iso-) oder linearen (n-) Produkten führen. Somit ist neben der Ausbeute die n-/iso-Selektivität ein wichtiger Parameter bei der Entwicklung neuer katalytischer Systeme für die Alkoxycarbonylierung.

Es ist bekannt, Monophosphinverbindungen als Liganden für die Alkoxycarbonylierung einzusetzen. Ein Beispiel hierfür ist die Alkoxycarbonylierung von Isopren mit Benzylalkohol in Gegenwart eines Pd-Komplexes. Bei dieser Reaktion wurden beispielsweise unter Verwendung des unter dem Handelsnamen cataCXium PtB erhältlichen Liganden N-Phenyl-2-(di-tert-butylphosphino)pyrrol gute Ausbeuten erzielt (Fang X. et al., Angew. Chem. Int. Ed., 2014, 53, 9030-9034). Allerdings erzielt dieser Ligand eine nur geringe Selektivität. Ähnliche heteroarylsubstituierte Monophosphinverbindungen, nämlich N-Phenyl-2-(di-phenyl-phosphino)pyrrol und N-Phenyl-2-(di-cyclohexyl-phosphino)pyrrol, wurden ebenfalls untersucht, erzielen aber nur geringe Ausbeuten bei der Alkoxycarbonylierung von Isopren mit Benzylalkohol (Fang X. *et al.,* aaO.).

In EP 0 386 834 A1 wird ein katalytisches System beschreiben, welches ein Metall der Gruppe VIII und ein Phosphin aufweist. Es werden des Weiteren ein Verfahren zur Herstellung des Phosphins, sowie der Einsatz des katalytischen Systems zur Carbonylierung von olefinisch ungesättigten Verbindungen beschrieben.

Die vorliegende Erfindung hat die Aufgabe, neue Liganden für die Alkoxycarbonylierung bereitzustellen, mit denen sich eine hohe Ausbeute und hohe n/iso-Selektivität erzielen lassen. Insbesondere sollen die Liganden für die Alkoxycarbonylierung von langkettigen ethylenisch ungesättigten Verbindungen, beispielsweise C₈-Olefinen geeignet sein.

Diese Aufgabe wird gelöst durch Monophosphinverbindungen gemäß Formel (**I**) 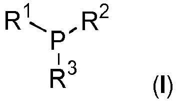 wobei
R¹ ausgewählt ist aus -(C₁-C₁₂)-Alkyl, -(C₃-C₁₂)-Cycloalkyl, -(C₃-C₁₂)-Heterocycloalkyl;
R² ausgewählt ist aus -(C₃-C₁₂)-Heterocycloalkyl, -(C₆-C₂₀)-Aryl, -(C₃-C₂₀)-Heteroaryl;
R³ für Imidazolyl steht;
und R¹, R² und R³ jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl, -CONH-(C₃-C₁₂)-Cycloalkyl,-CO-(C₁-C₁₂)-Alkyl, -CO-(C₃-C₁₂)-Cycloalkyl, -N-[(C₁-C₁₂)-Alkyl]₂, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

Die erfindungsgemäßen Monophosphinverbindungen eignen sich als Liganden für Pd-Komplexe, mit denen sich bei der Alkoxycarbonylierung einer Vielzahl ethylenisch ungesättigter Verbindungen hohe Ausbeuten erzielen lassen. Insbesondere eignen sich die erfindungsgemäßen Verbindungen zur Alkoxycarbonylierung von Ethen und langkettigen Olefinen wie 1-Octen.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, *n*-Butyl, *iso*-Butyl, *sec*-Butyl, *tert*-Butyl, *n*-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, *n*-Heptyl, 2-Heptyl, 3-Heptyl, 2-Ethylpentyl, 1-Propylbutyl, *n*-Octyl, 2-Ethylhexyl, 2-Propylheptyl, Nonyl, Decyl.

Die Erläuterungen zum Begriff (C₁-C₁₂)-Alkyl gelten insbesondere auch für die Alkylgruppen in -O-(C₁-C₁₂)-Alkyl, -S-(C₁-C₁₂)-Alkyl, -COO-(C₁-C₁₂)-Alkyl, -CONH-(C₁-C₁₂)-Alkyl, -CO-(C₁-C₁₂)-Alkyl und -N-[(C₁-C₁₂)-Alkyl]₂.

Der Begriff (C₃-C₁₂)-Cycloalkyl umfasst mono-, bi- oder tricyclische Kohlenwasserstoffgruppen mit 3 bis 12 Kohlenstoffatomen. Vorzugsweise handelt es sich dabei um (C₅-C₁₂)-Cycloalkyl.

Die (C₃-C₁₂)-Cycloalkylgruppen weisen vorzugsweise 3 bis 8, besonders bevorzugt 5 oder 6, Ringatome auf.

Geeignete (C₃-C₁₂)-Cycloalkylgruppen sind insbesondere Cyclopropyl, Cyclobutyl, Cyclopentyl, Cyclohexyl, Cycloheptyl, Cyclooctyl, Cyclododecyl, Cyclopentadecyl, Norbonyl, Adamantyl.

Die Erläuterungen zum Begriff (C₃-C₁₂)-Cycloalkyl gelten insbesondere auch für die Cycloalkylgruppen in -O-(C₃-C₁₂)-Cycloalkyl, -S-(C₃-C₁₂)-Cycloalkyl, -COO-(C₃-C₁₂)-Cycloalkyl, -CONH-(C₃-C₁₂)-Cycloalkyl, -CO-(C₃-C₁₂)-Cycloalkyl.

Der Begriff (C₃-C₁₂)-Heterocycloalkyl umfasst nichtaromatische, gesättigte oder teilweise ungesättigte cycloaliphatische Gruppen mit 3 bis 12 Kohlenstoffatomen, wobei eins oder mehrere der Ringkohlenstoffatome durch Heteroatome ersetzt sind. Die (C₃-C₁₂)-Heterocycloalkylgruppen weisen vorzugsweise 3 bis 8, besonders bevorzugt 5 oder 6, Ringatome auf und sind ggf. mit aliphatischen Seitenketten substituiert. In den Heterocycloalkylgruppen sind im Unterschied zu den Cycloalkylgruppen eins oder mehrere der Ringkohlenstoffatome durch Heteroatome oder heteroatomhaltige Gruppen ersetzt. Die Heteroatome oder die heteroatomhaltigen Gruppen sind vorzugsweise ausgewählt unter O, S, N, N(=O), C(=O), S(=O). Eine (C₃-C₁₂)-Heterocycloalkylgruppe im Sinne dieser Erfindung ist somit auch Ethylenoxid.

Geeignete (C₃-C₁₂)-Heterocycloalkylgruppen sind insbesondere Tetrahydrothiophenyl, Tetrahydrofuryl, Tetrahydropyranyl und Dioxanyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

Der Begriff (C₃-C₂₀)-Heteroaryl umfasst mono- oder polycyclische aromatische Kohlenwasserstoffreste mit 3 bis 20 Kohlenstoffatomen, wobei eins oder mehrere der Kohlenstoffatome durch Heteroatome ersetzt sind. Bevorzugte Heteroatome sind N, O und S. Die (C₃-C₂₀)-Heteroarylgruppen weisen 3 bis 20, bevorzugt 6 bis 14, besonders bevorzugt 6 bis 10 Ringatome auf. Somit ist beispielsweise Pyridyl im Rahmen dieser Erfindung ein C₆-Heteroarylrest, Furyl ist ein C₅-Heteroarylrest.

Geeignete (C₃-C₂₀)-Heteroarylgruppen sind insbesondere Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl.

Der Begriff Halogen umfasst insbesondere Fluor, Chlor, Brom und lod. Besonders bevorzugt sind Fluor und Chlor.
In einer Ausführungsform können die Reste R¹, R² und R³ jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl, -(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein.

In einer Ausführungsform können die Reste R¹, R² und R³ jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl substituiert sein.

In einer Ausführungsform können die Reste R¹, R² und R³ jeweils unabhängig voneinander mit einem oder mehreren Substituenten ausgewählt aus -(C₁-C₁₂)-Alkyl, -(C₃-C₁₂)-Cycloalkyl, -O-(C₁-C₁₂)-Alkyl, -O-(C₃-C₁₂)-Cycloalkyl, -(C₆-C₂₀)-Aryl substituiert sein.

In einer Ausführungsform können die Reste R¹, R² und R³ jeweils unabhängig voneinander mit einem oder mehreren Substituenten ausgewählt aus -(C₁-C₁₂)-Alkyl substituiert sein.

In einer Ausführungsform sind die Reste R¹, R² und R³ unsubstituiert.

In einer bevorzugten Ausführungsform steht R¹ für -(C₁-C₁₂)-Alkyl, vorzugsweise -(C₁-C₆)-Alkyl, besonders bevorzugt -(C₁-C₄)-Alkyl.

In einer bevorzugten Ausführungsform steht R² für -(C₆-C₂₀)-Aryl oder -(C₃-C₂₀)-Heteroaryl.

In einer Ausführungsform ist R², falls R² für einen Heteroarylrest steht, ausgewählt aus Heteroarylresten mit fünf bis zehn Ringatomen, vorzugsweise fünf oder sechs Ringatomen.

In einer Ausführungsform ist R², falls R² für einen Heteroarylrest steht, ausgewählt aus Heteroarylresten mit sechs bis zehn Ringatomen, vorzugsweise sechs Ringatomen.

In einer Ausführungsform ist R², falls R² für einen Heteroarylrest steht, ausgewählt aus Furyl, Thienyl, Pyrrolyl, Oxazolyl, Isoxazolyl, Thiazolyl, Isothiazolyl, Imidazolyl, Pyrazolyl, Furazanyl, Tetrazolyl, Pyridyl, Pyridazinyl, Pyrimidyl, Pyrazinyl, Benzofuranyl, Indolyl, Isoindolyl, Benzimidazolyl, Chinolyl, Isochinolyl, wobei die genannten Heteroarylreste wie oben beschrieben substituiert sein können.

In einer Ausführungsform ist R², falls R² für einen Heteroarylrest steht, ausgewählt aus Furyl, Thienyl, Pyrrolyl, Imidazolyl, Pyridyl, Pyrimidyl, Indolyl; insbesondere Pyrimidyl und Imidazolyl; wobei die genannten Heteroarylreste wie oben beschrieben substituiert sein können.

In einer Ausführungsform ist R², falls R² für einen Heteroarylrest steht, ausgewählt aus 2-Furyl, 2-Thienyl, 2-Pyrrolyl, 2-Imidazolyl, 2-Pyridyl, 2-Pyrimidyl, 2-Indolyl; insbesondere 2-Pyrimidyl und 2-Imidazolyl; wobei die genannten Heteroarylreste wie oben beschrieben substituiert sein können.

In einer Ausführungsform ist R² ausgewählt aus Phenyl, Pyrimidyl und Imidazolyl, vorzugsweise Phenyl, 2-Pyrimidyl und 2-Imidiazolyl, wobei die genannten Reste wie oben beschrieben substituiert sein können. Vorzugsweise ist R² ausgewählt aus Phenyl, 2-Pyrimidyl und N-Methyl-Imidazol-2-yl, wobei die genannten Reste nicht weiter substituiert sind.

In einer besonders bevorzugten Ausführungsform steht R³ für 2-Imidazolyl, wobei die genannten Reste wie oben beschrieben substituiert sein können. Insbesondere steht R³ für N-Methyl-imidazol-2-yl, wobei die genannten Reste nicht weiter subsituiert sind.

In einer Ausführungsform ist R¹ ausgewählt aus -(C₁-C₁₂)-Alkyl; R² ausgewählt aus Phenyl, 2-Pyrimidyl und 2-Imidazolyl; und R³ steht für 2-Imidazolyl; wobei die Reste R¹, R² und R³ jeweils wie oben beschrieben substituiert sein können.

In einer Ausführungsform weist die Verbindung die Struktur gemäß der Formeln (1) auf:

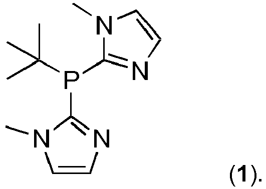

Die Erfindung betrifft weiterhin Komplexe umfassend Pd und eine erfindungsgemäße Monophosphinverbindung. Bei diesen Komplexen dient die erfindungsgemäße Monophosphinverbindung als Ligand für das Metallatom. Die Komplexe dienen beispielsweise als Katalysatoren für die Alkoxycarbonylierung. Mit den erfindungsgemäßen Komplexen lassen sich hohe Ausbeuten bei der Alkoxycarbonylierung einer Vielzahl unterschiedlicher ethylenisch ungesättigter Verbindungen erzielen.

Die erfindungsgemäßen Komplexe können außerdem weitere Liganden umfassen, die an das Metallatom koordinieren. Dabei handelt es sich beispielsweise um ethylenisch ungesättigte Verbindungen oder Anionen. Geeignete zusätzliche Liganden sind beispielsweise Styrol, Acetat-Anionen, Maleinimide (z.B. N-Methylmaleinimid), 1,4-Naphthochinon, Trifluoroacetat-Anionen oder Chloridanionen.

Die Erfindung betrifft weiterhin die Verwendung einer erfindungsgemäßen Monophosphinverbindung zur Katalyse einer Alkoxycarbonylierungsreaktion. Dabei kann die erfindungsgemäße Verbindung insbesondere als erfindungsgemäßer Metallkomplex eingesetzt werden.

Die Erfindung betrifft außerdem ein Verfahren umfassend die Verfahrensschritte:
a) Vorlegen einer ethylenisch ungesättigten Verbindung;
b) Zugabe einer erfindungsgemäßen Monophosphinverbindung und einer Verbindung, welche Pd umfasst,
   oder Zugabe eines erfindungsgemäßen Komplexes umfassend Pd und eine erfindungsgemäße Monophosphinverbindung;
c) Zugabe eines Alkohols;
d) Zuführen von CO;
e) Erwärmen des Reaktionsgemisches, wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform umfasst die ethylenisch ungesättigte Verbindung 2 bis 30 Kohlenstoffatome, bevorzugt 6 bis 22 Kohlenstoffatome, besonders bevorzugt 8 bis 12 Kohlenstoffatome, am meisten bevorzugt 8 Kohlenstoffatome.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten.

In einer Ausführungsform umfasst die ethylenisch ungesättigte Verbindung keine weiteren funktionellen Gruppen außer Kohlenstoff-Kohlenstoff-Doppelbindungen.

In einer besonders bevorzugten Ausführungsform handelt es sich bei der ethylenisch ungesättigten Verbindung um ein unfunktionalisiertes Alken mit mindestens einer Doppelbindung und 2 bis 30 Kohlenstoffatomen, bevorzugt 6 bis 22 Kohlenstoffatomen, weiter bevorzugt 8 bis 12 Kohlenstoffatomen, am meisten bevorzugt 8 Kohlenstoffatomen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure;
Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt.

Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren n-Butenen, Isobutan und n-Butan nach Abtrennung von Isobuten aus Raffinat I. Raffinat III ist ein Teil der beim Naphthacracken entstehenden C₄-Fraktion und besteht im Wesentlichen aus den isomeren n-Butenen und n-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch den erfindungsgemäßen Pd-Komplex katalysiert. Der Pd-Komplex kann dabei in Verfahrensschritt b) entweder als präformierter Komplex umfassend Pd und den erfindungsgemäßen Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

In dem Fall, dass der Katalysator *in situ* gebildet wird, kann der Ligand im Überschuss zugegeben werden, so dass im Reaktionsgemisch auch ungebundener Ligand vorliegt.

Auch im Falle des Komplexes, der gleich zu Beginn zugesetzt wird, kann zusätzlich weiterer Ligand zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂].

Vorzugsweise handelt es sich bei der Verbindung, welche Pd umfasst, um PdCl₂, Pd(acac)₂ oder Pd(OAc)₂. Besonders geeignet ist PdCl₂.

Der Alkohol im Verfahrensschritt c) kann verzweigt oder linear sein, cyclisch, alicyclisch, teilweise zyklisch oder aliphatisch und stellt insbesondere ein C₁- bis C₃₀-Alkanol dar. Es können Monoalkohole oder Polyalkohole verwendet werden.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl- , Amino-, Ether-, Thioether-, Aryl-, Heteroaryl- oder Silylgruppen, und/oder Halogensubstituenten enthalten.

In einer Ausführungsform umfasst der Alkohol keine weiteren funktionellen Gruppen außer Hydroxylgruppen.

Der Alkohol kann ungesättigte und aromatische Gruppen enthalten. Vorzugsweise handelt es sich jedoch um einen aliphatischen Alkohol.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol.

In einer Ausführungsform handelt es sich bei dem Alkohol um ein Alkanol mit einer oder mehrerer Hydroxylgruppen und 1 bis 30 Kohlenstoffatomen, bevorzugt 1 bis 22 Kohlenstoffatomen, besonders bevorzugt 1 bis 12 Kohlenstoffatomen, am meisten bevorzugt 1 bis 6 Kohlenstoffatomen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, Phenol, 2-Ethylhexanol, Isononanol, 2-Propylheptanol.

In einer bevorzugten Variante ist der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis der erfindungsgemäßen Monophosphinverbindung zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 3 mol-%, bezogen auf die Stoffmenge der in Schritt a) eingesetzten ethylenisch ungesättigten Verbindung.

### Beispiele

Im Folgenden wird die Erfindung anhand von Ausführungsbeispielen näher beschrieben.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795-1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Darstellung von Chlor-2-pyridyl-tert-butylphosphin (Vorstufe A)

Der Grignard für die Synthese von Chlor-2-pyridyl-t-butylphosphin wird nach der "Knochelmethode" mit Isopropylmagnesiumchlorid dargestellt (Angew. Chem. 2004, 43, 2222-2226). Die Aufarbeitung erfolgt nach der Methode von Budzelaar (Organometallics 1990, 9, 1222-1227).

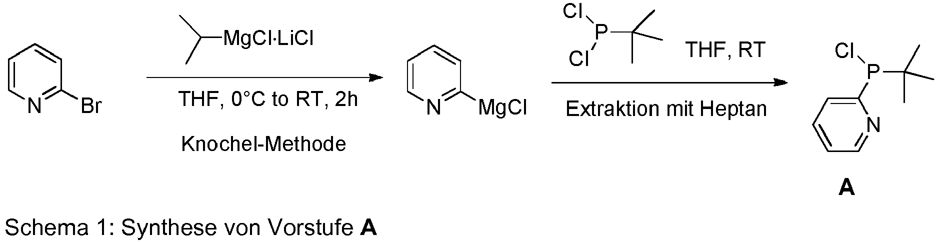

In einen 50 ml-Rundkolben mit Magnetrührer und Septum werden unter Argon 8,07 ml einer 1,3 M Isopropylmagnesium-chloridlösung (Knochel-Reagenz) gegeben und auf -15 °C gekühlt. Danach werden zügig 953.5 µl (10 mmol) 2-Bromopyridin zugetropft. Die Lösung wird sofort gelb. Man lässt auf -10 °C aufwärmen. Der Umsatz der Reaktion wird wie folgt bestimmt: man entnimmt ca. 100 µl Lösung und gibt sie in 1 ml einer gesättigten Ammoniumchloridlösung. Wenn die Lösung "sprudelt", dann hat sich noch nicht viel Grignard gebildet. Die wässrige Lösung wird mit einer Pipette Ether extrahiert und die organische Phase über Na₂SO₄ getrocknet. Von der etherischen Lösung wird ein GC aufgenommen. Wenn sich viel Pyridin gebildet hat im Vergleich zu 2-Bromopyridin, dann hat man hohe Umsätze. Bei -10 °C hat sich wenig umgesetzt. Nach Aufwärmen auf Raumtemperatur und 1-2 stündigen Rühren wird die Reaktionslösung braungelb. Ein GC-Test zeigt vollständigen Umsatz. Jetzt kann die Grignardlösung zu einer Lösung von 1,748 g (11 mmol) Dichloro-tert-butylphosphin in 10 ml THF, die vorher auf -15 °C gekühlt worden ist, mit einer Spritzenpumpe langsam zugetropft werden. Wichtig ist, dass die Dichloro-tert-butylphosphinlösung gekühlt wird. Bei Raumtemperatur würde man erhebliche Mengen an Dipyridyl-tert-butylphosphin erhalten. Es entsteht anfangs eine klare gelbe Lösung, die dann trübe wird. Man lässt auf Raumtemperatur aufwärmen und über Nacht Rühren. Laut GCMS hat sich viel Produkt gebildet. Man entfernt das Lösungsmittel im Hochvakuum und erhält einen weißlichen Feststoff, der braune Stellen enthält. Der Feststoff wird mit 20 ml Heptan suspendiert und der Feststoff wird im Ultraschallbad zerkleinert. Nach dem Absetzen lassen des weißen Feststoffes wird die Lösung dekantiert. Der Vorgang wird zwei Mal mit je 10-20 ml Heptan wiederholt. Nach dem Einengen der Heptanlösung im Hochvakuum wird diese unter Vakuum destilliert. Bei 4,6 mbar, 120 °C Ölbad und 98 °C Übergangstemperatur kann das Produkt destilliert werden. Man erhält 1,08 g eines farblosen Öls. (50%).
Analytische Daten: ¹H NMR (300 MHz, C₆D₆): δ 8,36 (m, 1H, Py), 7,67 (m, 1H, Py), 7,03-6,93 (m, 1H, Py), 6,55-6,46 (m, 1H, Py), 1,07 (d, J = 13,3 Hz, 9H, t-Bu).
¹³C NMR (75 MHz, C₆D₆): δ 162,9, 162,6, 148,8, 135,5, 125,8, 125,7, 122,8, 35,3, 34,8, 25,9 und 25,8.
³¹P NMR (121 MHz, C₆D₆) δ 97,9.
MS (EI) *m*:*z* (relative Intensität) 201 (M⁺,2), 147(32), 145 (100), 109 (17), 78 (8), 57,1 (17).

### Herstellung von Verbindung 1

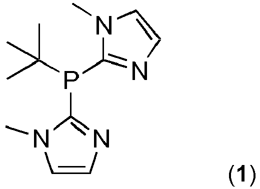

0,78 g (9,5 mmol) 1-Methylimidazol werden in einem 50 ml-Dreihalskolben mit Thermometer und Tropftrichter unter Argon eingewogen und in 10 ml THF gelöst. Dann gibt man 1,6 ml TMEDA zu der Lösung. Das Gemisch wird dann auf -78 °C heruntergekühlt. Danach werden 6 ml 1,6 N n-Butyllithium in Hexan mittels Tropftrichter zugetropft. Den 50 ml-Kolben mit der Reaktionsmischung lässt man 30 min bei Raumtemperatur rühren. Anschließend löst man 1,5 g tert-Butyldichlorphosphin in 20 ml THF. Man tropft dann das 1-Methylimidazol-BuLi-Gemisch bei -78 °C zum tert-Butyldichlorphosphin. Danach wird auf Raumtemperatur erwärmt. Es fällt ein Produkt aus. Die Suspension wird filtriert und der Rückstand wird in Wasser gelöst und anschließend dreimal mit Dichlormethan gewaschen. Die organische Phase wird mit Na₂SO₄ getrocknet und dann das Lösungsmittel im Vakuum entfernt. Den Rückstand löst man mit 5 ml Dichlormethan und überschichtet mit 20 ml Diethylether. Es kristallisiert das Produkt. Das Produkt konnte in 0,8 g erhalten werden.
Reinheit (NMR)= 98%,
³¹P NMR (CD₂Cl₂, 121 MHz)= -32,25 ppm,
¹³C NMR(CD₂Cl₂, 75 MHz) = 144 s, 130,2 d (J_{PC} = 3,7 Hz), 123,8 s, 34,2 d, (J_{PC} = 11,7 Hz), 25,9 d, (J_{PC} = 14,3 Hz)
¹H NMR (CD₂Cl₂, 300 MHz,): 7,04, d, (J = 1 Hz, 1H), 6,94 dd (J = 1 Hz, J = 1,5 Hz, 1H), 3,4 s (6H), 1,2 d (J = 14,6 Hz, 9H)
HRMS: berechnet für C₁₂H₁₉N₄P: 251,14201, gefunden: 251,14206

### Herstellung von 2-(tert-Butyl(phenyl)phosphino)pyridin (Verbindung 2)

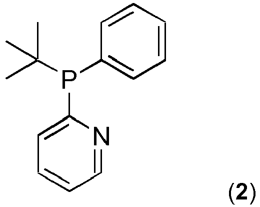

3,4 g (16,8 mmol) 2-(tert-Butylchlorphosphino)pyridin (Vorstufe **A**) werden unter Argon in einem 100 ml-Dreihalskolben versehen mit Tieftemperaturthermometer und Magnetrührer in 50 ml absolutem Diethylether gelöst. Es wird auf -78 °C heruntergekühlt. Bei dieser Temperatur werden innerhalb von 10 Minuten 10 ml einer 1,8 N Phenyllithiumlösung (in Dibutylether) mittels eines Tropftrichters zugesetzt. Es wird 10 Minuten bei dieser Temperatur gerührt und anschließend auf Raumtemperatur erwärmt und noch eine weiter halbe Stunde gerührt. Diese Lösung wird dreimal mit 10 ml entgastem Wasser gewaschen. Die organische Phase wird nun im Feinvakuum von 10⁻¹ Torr destilliert. Das Produkt wird bei diesem Druck bei 130 °C als klare Flüssigkeit in hoher Reinheit von größer 97 % (NMR) erhalten. Die Ausbeute beträgt 3,85 g (93%).

### Analytik:

³¹P (Aceton-d₆, 121 MHz), 16,31 s,
¹³C (75 MHz, Acetond₆, 165,1 (d, J_{PC} = 10,5 Hz), 150,3 (d, J_{PC} = 5 Hz), 137,3 s, 137,0 s, 136,7 s, 135,9 d, 135,9 (d, J_{PC} = 7,6 Hz), 131,1 s, 130,6 s, 130,2 s, 128,9 (d, J_{PC} = 8 Hz), 122,9 s, 32,1 (d, J_{PC} = 13,1 Hz), 28,5 (d, J_{PC} = 13,7 Hz),
¹H (Aceton-d₆, 300 MHz): 8,74 (dm, J = 4,7 Hz), 7,7-7,6 m (2 H), 7,4-7,3 (m, 3 H), 7,28-7,23 (m, 1 H), 1,2 (d, J = 12,6 Hz, 9 H)
MS (EI, 70 eV): *m*/*z* (%), 243 (M+, 17), 203 (65), 187 (78), 156 (6), 126(8), 109(100), 78(11), 57(11), HRMS(EI), berechnet für C15H18N1P1: 243.11714, gefunden: 243,11753

### Weitere Liganden

Die folgenden Vergleichsverbindungen sind kommerziell verfügbar.

| | |
|---|---|
| 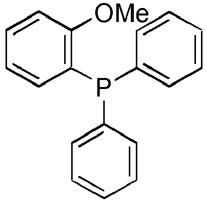 | **3** (VB) |
| 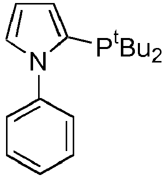 | **10** (VB) |

| | |
|---|---|
| VB: Vergleichsbeispiel | |

### Alkoxycarbonylierungsversuche

### Allgemeine Versuchsbeschreibung für Reaktionen im Batchversuch

Die entsprechenden Mengen an Substrat, Palladiumsalz, Säure und Alkohol werden unter Argon und Magnetrührung in einem 50 ml-Schlenkgefäß vermischt.

Ein 100 ml Stahlautoklav der Firma Parr versehen mit einem Gaseinlass und einem Gasauslassventil, einem digitalem Druckaufnehmer, einem Temperaturfühler und einem Kugelhahn sowie einer eingebauten Kapillare zur Probennahme wird dreimal mittels Vakuum und Argonspülung von Sauerstoff befreit. Anschließend wird die Reaktionslösung aus dem Schlenkgefäß mittels einer Kapillare in den Autoklaven im Argongegenstrom durch den Kugelhahn befüllt. Anschließend wird entweder bei Raumtemperatur die entsprechende Menge an CO aufgepresst und dann auf Reaktionstemperatur hochgeheizt (Reaktionen, die nicht unter konstantem Druck gefahren werden) oder es wird erst auf Reaktionstemperatur hochgeheizt und dann wird das CO über eine Bürette, die mittels eines Druckminderers mit dem Autoklaven verbunden ist, aufgepresst. Diese Bürette wird dann noch auf ca. 100 bar mit CO befüllt und liefert während der Reaktion das benötigte CO bei einem konstanten Druck nach. Diese Bürette hat ein Totvolumen von ca. 30 ml und ist mit einem digitalen Druckaufnehmer versehen. Dann wird die Reaktion bei der benötigten Temperatur die entsprechende Zeit unter Rührung durchgeführt. Hierbei werden mittels einer Software (Specview der Firma SpecView Corporation) und einem Prozesscontroller der Firma Parr 4870 sowie einem 4875 Powercontroler Daten über den Druckverlauf im Autoklaven und in der Gasbürette aufgezeichnet. Aus diesen werden Exceltabellen generiert, aus denen später Diagramme erstellt werden, die Gasverbräuche und damit Umsätze über die Zeit darstellen. Falls benötigt, werden über die Kapillare über die GC Proben gesammelt und analysiert. Hierzu wird vor der Reaktion eine geeignete exakte Menge (2-10 ml) Isooctan als interner Standard mit in das Schlenkgefäß gegeben. Diese geben auch Auskunft über den Reaktionsverlauf. Am Ende der Reaktion wird der Autoklav auf Raumtemperatur heruntergekühlt, der Druck vorsichtig abgelassen, falls nötig Isooctan als interner Standard hinzugefügt und eine GC-Analyse bzw. bei neuen Produkten auch eine GC MS Analyse durchgeführt.

### Allgemeine Versuchsvorschrift für Autoklavenversuche in Glasvials

Es wird ein 300ml Parrreaktor verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt. Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur erhitzt und die Reaktion die entsprechende Zeit durchgeführt. Anschließend wird auf Raumtemperatur heruntergekühlt und der Druck langsam abgelassen. Anschließend wird der Autoklav mit Stickstoff gespült. Die Vials werden dem Autoklaven entnommen und mit einer definierten Menge eines geeigneten Standards versetzt. Es erfolgt eine GC Analyse mit deren Ergebnissen Ausbeuten und Selektivitäten bestimmt wurden.

### Analytik:

### Analytik Methanol

Methanol wurde in einer Lösungsmitteltrocknungsanlage vorbehandelt: Pure Solv MD-/ Lösungsmittel Purification System, Firma Innovative Technology Inc. One Industrial Way, Amesbury MA 01013

### Wasserwerte:

Bestimmt mit Karl Fischer Tritration: TitraLab 580-TIM580, Firma Radiometer Analytical SAS (Karl- Fischer Titration), Wassergehalt: Messbereiche, 0,1-100% w/w, gemessener Wassergehalt: 0,13889%

### Eingesetzt wurden:

Technisches Methanol Applichem: Nr. A2954,5000, Chargennummer: LOT: 3L005446 Wassergehalt max. 1 %
Methanol Acros Organics (über Molsieb): Wassergehalt 0,005 %, Codenummer: 364390010, Chargennummer: LOT 1370321

### Methoxycarbonylierung von Ethen

Ein 50 ml Schlenkgefäß wurde mit Pd(acac)₂ (6,53 mg, 0,04 mol%), Ligand (0,16 mol%), Ethen (1,5 g, 53 mmol), 20ml Methanol und para-Toluolsulfonsäure (PTSA, 61 mg, 0,6 mol%) beladen. Das Reaktionsgemisch wurde mittels einer Kapillare im Argongegenstrom in einen 100 ml-Stahl-Autoklaven wie oben beschrieben überführt. Der CO-Druck wurde auf 40 bar eingestellt. Die Reaktion lief 3 Stunden bei 80°C. Nach Abschluss der Reaktion wurde der Autoklav auf Raumtemperatur abgekühlt und vorsichtig entspannt. Isooctan (100 µl) wurde als interner GC-Standard zugesetzt. Ausbeute und Regioselektivität wurden mittels GC bestimmt.

Die Ergebnisse sind in der nachfolgenden Tabelle dargestellt.

| Ligand | Ausbeute |
|---|---|
| **1** | 30% |
| **2** (VB) | 14% |
| **3** (VB) | 3% |
| **10** (VB) | 0% |

| | |
|---|---|
| VB: Vergleichsbeispiel | |

Dieses Beispiel zeigt, dass die erfindungsgemäßen Liganden **1** deutlich bessere Ausbeuten bei der Methoxycarbonylierung von Ethen erzielen als die Vergleichsliganden **2, 3** und **10.**

## Patentansprüche

1. Verbindung gemäß Formel (**I**) 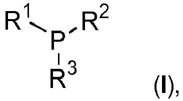 wobei
R¹ ausgewählt ist aus -(C₁-C₁₂)-Alkyl, -(C₃-C₁₂)-Cycloalkyl, -(C₃-C₁₂)-Heterocycloalkyl;
R² ausgewählt ist aus -(C₃-C₁₂)-Heterocycloalkyl, -(C₆-C₂₀)-Aryl, -(C₃-C₂₀)-Heteroaryl;
R³ für Imidazolyl steht;
und R¹, R² und R³ jeweils unabhängig voneinander mit einem oder mehreren Substituenten ausgewählt aus -(C₁-C₁₂)-Alkyl, -(C₃-C₁₂)-Cycloalkyl, -(C₃-C₁₂)-Heterocycloalkyl, -O-(C₁-C₁₂)-Alkyl, -O-(C₁-C₁₂)-Alkyl-(C₆-C₂₀)-Aryl, -O-(C₃-C₁₂)-Cycloalkyl, -S-(C₁-C₁₂)-Alkyl, -S-(C₃-C₁₂)-Cycloalkyl, -COO-(C₁-C₁₂)-Alkyl, -COO-(C₃-C₁₂)-Cycloalkyl, -CONH-(C₁-C₁₂)-Alkyl,-CONH-(C₃-C₁₂)-Cycloalkyl, -CO-(C₁-C₁₂)-Alkyl, -CO-(C₃-C₁₂)-Cycloalkyl, -N-[(C₁-C₁₂)-Alkyl]2, -(C₆-C₂₀)-Aryl, -(C₆-C₂₀)-Aryl-(C₁-C₁₂)-Alkyl, -(C₆-C₂₀)-Aryl-O-(C₁-C₁₂)-Alkyl,-(C₃-C₂₀)-Heteroaryl, -(C₃-C₂₀)-Heteroaryl-(C₁-C₁₂)-Alkyl, -(C₃-C₂₀)-Heteroaryl-O-(C₁-C₁₂)-Alkyl, -COOH, -OH, -SO₃H, -NH₂, Halogen substituiert sein können.

2. Verbindung nach Anspruch 1,
wobei R¹ für -(C₁-C₁₂)-Alkyl steht.

3. Verbindung nach einem der Ansprüche 1 bis 2,
wobei R² ausgewählt ist aus -(C₆-C₂₀)-Aryl und -(C₃-C₂₀)-Heteroaryl.

4. Verbindung nach einem der Ansprüche 1 bis 3,
wobei R² ausgewählt ist aus Phenyl, Pyrimidyl und Imidazolyl.

5. Verbindung nach einem der Ansprüche 1 bis 4,
wobei R¹, R² und R³ jeweils unabhängig voneinander mit einem oder mehreren Substituenten ausgewählt aus -(C₁-C₁₂)-Alkyl substituiert sein können.

6. Verbindung nach einem der Ansprüche 1 bis 5,
gemäß der Formel (**1**)

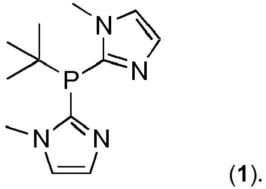

7. Komplex umfassend Pd und eine Verbindung nach einem der Ansprüche 1 bis 6.

8. Verfahren umfassend die Verfahrensschritte:
a) Vorlegen einer ethylenisch ungesättigten Verbindung;
b) Zugabe einer Verbindung nach einem der Ansprüche 1 bis 6 und einer Verbindung, welche Pd umfasst,
oder Zugabe eines Komplexes nach Anspruch 7;
c) Zugabe eines Alkohols;
d) Zuführen von CO;
e) Erwärmen des Reaktionsgemisches, wobei die ethylenisch ungesättigte Verbindung zu einem Ester umgesetzt wird.

9. Verfahren nach Anspruch 8,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, di-n-Buten, oder Mischungen davon.

10. Verfahren nach einem der Ansprüche 8 bis 9,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

11. Verfahren nach einem der Ansprüche 8 bis 10,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

12. Verfahren nach einem der Ansprüche 8 bis 11,
wobei der Alkohol in Verfahrensschritt c) ein aliphatischer Alkohol ist.

13. Verwendung einer Verbindung gemäß einem der Ansprüche 1 bis 6 oder eines Komplexes gemäß Anspruch 7 zur Katalyse einer Alkoxycarbonylierungsreaktion.

## Claims

1. Compound according to formula (**I**) 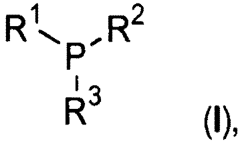 where
R¹ is selected from among -(C₁-C₁₂)-alkyl, -(C₃-C₁₂)-cycloalkyl, -(C₃-C₁₂)-heterocycloalkyl;
R² is selected from among -(C₃-C₁₂)-heterocycloalkyl, -(C₆-C₂₀)-aryl, -(C₃-C₂₀)-heteroaryl;
R³ is imidazolyl;
and R¹, R² and R³ can each be substituted independently of one another by one or more substituents selected from among -(C₁-C₁₂)-alkyl, -(C₃-C₁₂)-cycloalkyl, -(C₃-C₁₂)-heterocycloalkyl, -O-(C₁-C₁₂)-alkyl, -O-(C₁-C₁₂)-alkyl-(C₆-C₂₀)-aryl, -O-(C₃-C₁₂)-cycloalkyl, -S-(C₁-C₁₂)-alkyl, -S-(C₃-C₁₂)-cycloalkyl, -COO-(C₁-C₁₂)-alkyl, -COO-(C₃-C₁₂)-cycloalkyl, -CONH-(C₁-C₁₂)-alkyl, -CONH-(C₃-C₁₂)-cycloalkyl, -CO-(C₁-C₁₂)-alkyl, -CO-(C₃-C₁₂)-cycloalkyl, -N-[(C₁-C₁₂)-alkyl]₂, -(C₆-C₂₀)-aryl, -(C₆-C₂₀)-aryl-(C₁-C₁₂)-alkyl, -(C₆-C₂₀)-aryl-O-(C₁-C₁₂)-alkyl, -(C₃-C₂₀)-heteroaryl, -(C₃-C₂₀)-heteroaryl-(C₁-C₁₂)-alkyl, -(C₃-C₂₀)-heteroaryl-O-(C₁-C₁₂)-alkyl, -COOH, -OH, -SO₃H, -NH₂, halogen.

2. Compound according to Claim 1,
wherein R¹ is -(C₁-C₁₂)-alkyl.

3. Compound according to either of Claims 1 and 2,
wherein R² is selected from among -(C₆-C₂₀)-aryl and -(C₃-C₂₀)-heteroaryl.

4. Compound according to any of Claims 1 to 3,
wherein R² is selected from among phenyl, pyrimidyl and imidazolyl.

5. Compound according to any of Claims 1 to 4,
wherein R¹, R² and R³ can each be substituted independently of one another by one or more substituents selected from among -(C₁-C₁₂)-alkyl.

6. Compound according to any of Claims 1 to 5, according to the formula (**1**)

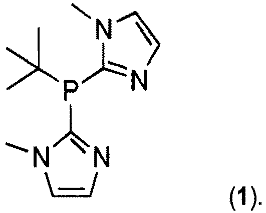

7. Complex comprising Pd and a compound according to any of Claims 1 to 6.

8. Process comprising the process steps:
a) initial charging of an ethylenically unsaturated compound;
b) addition of a compound according to any of Claims 1 to 6 and a compound comprising Pd, or addition of a complex according to Claim 7;
c) addition of an alcohol;
d) introduction of CO;
e) heating of the reaction mixture, resulting in the ethylenically unsaturated compound being converted into an ester.

9. Process according to Claim 8,
wherein the ethylenically unsaturated compound is selected from among ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene and mixtures thereof.

10. Process according to either of Claims 8 and 9,
wherein the compound comprising Pd in process step b) is selected from among palladium dichloride, palladium(II) acetylacetonate, palladium(II) acetate, dichloro(1,5-cyclooctadiene)palladium(II), bis(dibenzylideneactone)palladium, bis(acetonitrile)dichloropalladium(II), (cinnamyl)palladium dichloride.

11. Process according to any of Claims 8 to 10,
wherein the alcohol in process step c) is selected from among methanol, ethanol, 1-propanol, 1-butanol, 1-pentanol, 1-hexanol, 2-propanol, *tert-*butanol, 3-pentanol, cyclohexanol, phenol and mixtures thereof.

12. Process according to any of Claims 8 to 11,
wherein the alcohol in process step c) is an aliphatic alcohol.

13. Use of a compound according to any of Claims 1 to 6 or a complex according to Claim 7 for catalysis of an alkoxycarbonylation reaction.

## Revendications

1. Composé selon la formule (I) 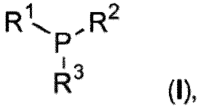 dans laquelle
R¹ est choisi parmi alkyle en (C₁-C₁₂), cycloalkyle en (C₃-C₁₂), hétérocycloalkyle en (C₃-C₁₂) ;
R² est choisi parmi hétérocycloalkyle en (C₃-C₁₂), aryle en (C₆-C₂₀), hétéroaryle en (C₃-C₂₀) ;
R³ représente imidazolyle ;
et R¹, R² et R³ peuvent chacun indépendamment les uns des autres être substitués avec un ou plusieurs substituants choisis parmi : alkyle en (C₁-C₁₂), cycloalkyle en (C₃-C₁₂), hétérocycloalkyle en (C₃-C₁₂), -O-alkyle en (C₁-C₁₂), -O-alkyle en (C₁-C₁₂)-aryle en (C₆-C₂₀), -O-cycloalkyle en (C₃-C₁₂), -S-alkyle en (C₁-C₁₂), -S-cycloalkyle en (C₃-C₁₂), -COO-alkyle en (C₁-C₁₂), -COO-cycloalkyle en (C₃-C₁₂), -CONH-alkyle en (C₁-C₁₂), -CONH-cycloalkyle en (C₃-C₁₂), -CO-alkyle en (C₁-C₁₂), -CO-cycloalkyle en (C₃-C₁₂), -N-[alkyle en (C₁-C₁₂)]₂, aryle en (C₆-C₂₀), aryle en (C₆-C₂₀)-alkyle en (C₁-C₁₂), aryle en (C₆-C₂₀) -O-alkyle en (C₁-C₁₂), hétéroaryle en (C₃-C₂₀), hétéroaryle en (C₃-C₂₀)-alkyle en (C₁-C₁₂), hétéroaryle en (C₃-C₂₀) -O-alkyle en (C₁-C₁₂), -COOH, -OH, -SO₃H, -NH₂, halogène.

2. Composé selon la revendication 1, dans lequel R¹ représente alkyle en (C₁-C₁₂).

3. Composé selon l'une quelconque des revendications 1 à 2, dans lequel R² est choisi parmi aryle en (C₆-C₂₀) et hétéroaryle en (C₃-C₂₀) .

4. Composé selon l'une quelconque des revendications 1 à 3, dans lequel R² est choisi parmi phényle, pyrimidyle et imidazolyle.

5. Composé selon l'une quelconque des revendications 1 à 4, dans lequel R¹, R² et R³ peuvent chacun indépendamment les uns des autres être substitués avec un ou plusieurs substituants choisis parmi alkyle en (C₁-C₁₂).

6. Composé selon l'une quelconque des revendications 1 à 5, selon la formule (1)

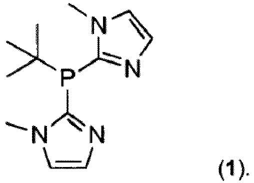

7. Complexe comprenant Pd et un composé selon l'une quelconque des revendications 1 à 6.

8. Procédé comprenant les étapes de procédé suivantes :
a) le chargement d'un composé éthyléniquement insaturé ;
b) l'ajout d'un composé selon l'une quelconque des revendications 1 à 6 et d'un composé qui comprend Pd,
ou l'ajout d'un complexe selon la revendication 7 ;
c) l'ajout d'un alcool ;
d) l'introduction de CO ;
e) le chauffage du mélange réactionnel, le composé éthyléniquement insaturé étant transformé en un ester.

9. Procédé selon la revendication 8, dans lequel le composé éthyléniquement insaturé est choisi parmi l'éthène, le propène, le 1-butène, le *cis-* et/ou *trans-*2-butène, l'iso-butène, le 1,3-butadiène, le 1-pentène, le *cis-* et/ou *trans*-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

10. Procédé selon l'une quelconque des revendications 8 à 9, dans lequel le composé qui comprend Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).

11. Procédé selon l'une quelconque des revendications 8 à 10, dans lequel l'alcool à l'étape de procédé c) est choisi parmi le méthanol, l'éthanol, le 1-propanol, le 1-butanol, le 1-pentanol, le 1-hexanol, le 2-propanol, le *tert*-butanol, le 3-pentanol, le cyclohexanol, le phénol ou leurs mélanges.

12. Procédé selon l'une quelconque des revendications 8 à 11, dans lequel l'alcool à l'étape de procédé c) est un alcool aliphatique.

13. Utilisation d'un composé selon l'une quelconque des revendications 1 à 6 ou d'un complexe selon la revendication 7 pour la catalyse d'une réaction d'alcoxycarbonylation.
